# EUROPÄISCHE PATENTSCHRIFT

(11) **EP 1 572 018 B1**
(45) Veröffentlichungstag und Bekanntmachung des Hinweises auf die Patenterteilung: **09.03.2011**
(21) Anmeldenummer: 03809739.0
(22) Anmeldetag: 28.10.2003
(51) Int. Cl.: A61B 17/86

(54) **KNOCHENNAGEL ZUM VERSORGEN VON FRAKTUREN**
FRACTURE PIN
CLOU PERMETTANT DE REPARER DES FRACTURES OSSEUSES

(30) Priorität: 29.10.2002 DE 20216843 U
(43) Veröffentlichungstag der Anmeldung: 14.09.2005
(73) Patentinhaber: Tantum AG, 24537 Neumünster (DE)
(72) Erfinder: CAPOUSEK, Milan, A-4073 Wilhering (AT); HARDER, Hans, Erich, 24253 Probsteierhagen (DE)
(74) Vertreter: Wenzel & Kalkoff
(86) Internationale Anmeldenummer: PCT/EP2003/011985
(87) Internationale Veröffentlichungsnummer: WO 2004/039271

(56) Entgegenhaltungen:
- EP-A- 0 551 588
- EP-A1- 0 682 917
- GB-A- 2 274 993
- US-A- 5 472 444
- US-A- 5 693 055

## Beschreibung

Die Erfindung betrifft einen Knochennagel zum Versorgen von Frakturen eines mehrere Segmente aufweisenden Knochens, nämlich von Frakturen des proximalen Kopfteils des Humerus, wobei der Knochennagel einen stabförmigen, insbesondere rohrförmigen, Stützkörper aufweist.

Derartige Knochennägel werden zur Versorgung von Frakturen des Humeruskopfes eingesetzt. Die Nägel dienen dazu, die einzelnen Bruchstücke zu fixieren, um ein Zusammenwachsen der Bruchstücke in der gewünschten und gewählten Position zu ermöglichen. Der Oberarmknochen (lateinisch: Humerus) weist drei wesentliche Knochensegmente auf, nämlich an einem proximalen Ende das Kopfteil (proximales Kopfteil), an einem distalen Ende das Fußteil sowie einen den Kopf mit dem Fuß verbindenden Schaft. Bekannte Knochennägel werden entsprechend den Bruchlinien in den zu stützenden Knochen eingesetzt. Dabei erstreckt sich der Knochennagel über mindestens zwei Knochensegmente. Bei einer Fraktur des Humeruskopfes erstreckt sich der Knochennagel vom Kopfteil bis in den Schaft. Bei einer Fraktur des Humerusfusses erstreckt sich der Knochennagel vom Fußteil bis in den Schaft.

Die bekannten Nägel weisen den Nachteil auf, daß sie eine große Traumatisierung des Knochens bewirken, da sie sich über mehrere Segmente des Humerus erstrecken, obwohl üblicherweise lediglich ein Segment zu versorgen ist. Des weiteren ist eine zusätzliche Stützung durch zusätzliche Befestigungselemente erforderlich, was zu einer weiteren Traumatisierung des Knochens führt (zum Beispiel US 5 472 444). Aus GB 2 274 993 A ist eine Einrichtung bekannt, die zum Versorgen einer einfachen subkapitalen Humerusfraktur eingerichtet ist. Ein nur an seinen Enden DUrchgangsbohrungen aufweisender Nagel nimmt in den Bohrungen Befestigungselemente auf, die nach distal relativ weit in den Humerusschaft hinein zu erstrecken sind Teil 1a in Fig. 12 ist ein Teil eines zweiteiligen Humerus-Knochennagels zum Versorgen von Frakturen des distalen Fußteils des Humerus (condylus humeri). Der Teil 1a und ein Teil 1b des Humerus-Knochennagels bilden einen stabförmigen Stützkörper, der als sich im Wesentlichen linear erstreckender Kurznagel mit medialem Ende und lateralem Ende derart kurz ausgebildet ist, dass er sich in den Humerus eingeführtem Zustand von dem medialen Ende zu dem lateralen Ende lediglich über das distale Ende des Humerusfußteils erstreckt. Der Teil 1 a weist an seinem dem Teil 1b zugewandten Ende ein Außengewinde zum Einschrauben in ein Innengewinde des Teils 1b auf, um Winkel zwischen Ebenen von Durchgangsbohrungen jeweils in den Teilen 1a und 1b zu justieren. Ein Abschnitt des Teils 1a sowie ein Abschnitt des Teils 1b weisen jeweils zwischen dem Ende mit dem Außengewinde bzw. mit dem Innengewinde und dem anderen Ende mindestens eine der Durchgangsbohrungen zur Aufnahme eines stiftartigen Befestigungselements auf. Jede Durchgangsbohrung kann derart ausgebildet sein, dass ein korrespondierendes, durch die Durchgangsbohrung hindurchgesetztes Befestigungselement in seiner linearen Bewegung begrenzt ist. US 5 693 055 offenbart eine andere Einrichtung zum Versorgen einer einfachen Querfraktur im proximalen Humerus zwischen Metaphysis und Diaphysis. Eine einfache Ankerschraube wird als Kompres-sionsschraube genutzt, um Frakturteile der subkapitalen Fraktur aneinander zu pressen. Aus DE 92 00 328 geht ein Verriegelungsnagel zum Versorgen von Frakturen des proximalen Femurs hervor. Ein solcher Nagel, der relativ kurz ausgeführt sein soll, ist so eingerichtet, dass er im Wesentlichen in der Markhöhle des Femurschafts außerhalb des Femurkopfes zu stützen und zu fixieren ist.

EP 0 682 917 A1 offenbart eine Schraube zum Zusammenhalten von Axis und Dens, die ein Außengewinde und Querbohrungen aufweist.

Es ist daher Aufgabe der vorliegenden Erfindung, einen Humerus-Knochennagel zu schaffen, der eine gezielte Versorgung von Kopffrakturen des Humerus und Stützung der Knochenfragmente bei gleichzeitig geringer Traumatisierung des Humerus gewährleistet. Insbesondere soll ein solcher Humerus-Knochennagel mit wenigstens einem Befestigungselement hinsichtlich einfacher Operations-Technik in günstiger Weise, insbesondere in geeigneter Sortierung zur Verfügung stehen.

Die Aufgabe wird durch die Merkmale des Anspruchs 1 überraschend gelöst. Der Humerus-Knochennagel bzw. der Stützkörper ist als Kurznagel derart ausgebildet, daß er sich in in den Humerus eingeführtem Zustand über ein einziges Knochensegment, nämlich den Humerus-Kopfteil erstreckt. Entgegen der bisherigen Lehre ist die Versorgung einer Fraktur des Humeruskopfes mit dem erfindungsgemäßen Kurznagel, der sich im Wesentlichen linear erstreckt, vollständig ausreichend, und zwar ausschließlich in dem betroffenen Knochensegment, so daß eine Schwächung des Knochens durch Befestigungselemente in weiteren, nicht von der Fraktur betroffenen Segmenten verhindert wird.

Gemäß der Erfindung weist der für eine Kopffraktur des Humerus vorgesehene Knochennagel ein proximales Ende auf, an dem er mit einem Außengewinde versehen ist. Durch das Gewinde ist eine zusätzliche Fixierung und Stützung innerhalb des Knochens, insbesondere im festen Randbereich des Knochens (der Compacta), gewährleistet, so daß der Knochennagel auch größeren Belastungen ausgesetzt werden kann. Der Knochennagel hat daher auch ohne zusätzliche Befestigungselemente festen Halt im Knochen.

Erfindungsgemäß weist der Stützkörper einen Mittelabschnitt auf, in dessen Bereich mindestens eine Durchgangsbohrung vorgesehen ist. Jede Durchgangsbohrung ermöglicht den Einsatz von zusätzlichen Befestigungselementen. Damit ist eine weitere zusätzliche Sicherung des Knochennagels innerhalb des Knochens gewährleistet.

Vorzugsweise ist das Außengewinde mit einer Steigung versehen, die zum einen eine ausreichende Halterung und Fixierung im Knochen gewährleistet, d.h. eine möglichst große Steigung, die ausreichend Spanraum zwischen den Gewindegängen zur Aufnahme von Knochensubstanz beläßt, und zum anderen eine Feinjustierung, d.h. eine möglichst kleine Steigung zuläßt, so daß bei einer Drehung lediglich ein geringer Vorschub erzielt wird. Die erfindungsgemäß abgestimmte Steigung des Gewindes kombiniert quasi zwei gegenläufige Forderungen in besonders vorteilhafter Weise. Es ist gefunden worden, daß Steigungen im Bereich von 1 bis 2,5 besonders günstig sind, wobei sich eine Steigung von 1,75 als besonders vorteilhaft erweist.

Das Außengewinde ist selbstschneidend ausgebildet, wodurch ein Vorschneiden des Gewindes, was zu einer Traumatisierung des Knochens führt, vermieden werden kann.

Gemäß vorteilhafter Weiterbildung der Erfindung weist der für eine Kopffraktur des Humerus vorgesehene Knöchennagel ein distales Ende des Stürzkörpers auf, an dem eine Schräge vorgesehen ist. Der Winkel der Schräge ist an die Kontur des Knochens derart angepaßt, daß der Knochennagel in eingeführtem bzw. eingesetztem Zustand mit seinem distalen Ende wenigstens nahezu bündig mit dem Knochen abschließt. Dadurch wird verhindert, daß ein Abschnitt des Knochennagels über die Außenkontur des Knochens hinausragt, was Verhaken oder dergleichen und damit verbundener Verletzungsgefahr wirksam verhindert. Der Stützkörper ist in einem Humeruskopf insbesondere im Fall üblicher Frakturmuster (Frakturlinien und -flächen) derart orientiert, daß er sich, ausgehend vom insbesondere mit der Schräge versehenen distalen Ende in den medialen Bereich des Humeruskopfes hinein erstreckt.

Zweckmäßig sind im Bereich des Mittelabschnittes des durch den Kurznagel zur Versorgung von Kopffrakturen des Humerus gebildeten Stützkörpers wenigstens drei und insbesondere vier Durchgangsbohrungen angeordnet und ausgebildet. Vorteilhaft bilden drei nach proximal am Mittelabschnitt liegende Durchgangsbohrungen solche, die Frakturendstellen durchsetzende Befestigungselemente aufnehmern. Eine oder mehrere nach distal gelegene Durchgangsbohrungen am Mittelabschnitt können zur Aufnahme von einem bzw. mehreren Befestigungselementen im Bereich des chirurgischen Halses des Humeruskopfes ausgebildet sein.

Vorteilhafterweise weist wenigstens eine Durchgangsbohrung an mindestens einer ihrer Endseiten einen Absatz auf, derart, daß ein korrespondierendes, durch die Durchgangsbohrung hindurchgesetztes stiftartiges Befestigungselement in seiner in Achsrichtung linearen Bewegung begrenzt ist. Dadurch wird bewirkt, daß das Befestigungselement selbst dann seine Stützfunktion wahrnehmen kann, wenn der Halt im Knochen (in der Spongiosa und/oder der Compacta) selbst nicht mehr gewährleistet ist, da ein Herausrutschen aus der Bohrung durch den Absatz verhindert wird.

Um wenigstens einen erfindungsgemäßen Knochen-Kurmagel mit wenigstens einem Befestigungselement zur Erleichterung der Operations-Technik bereitzuhalten, sieht die Erfindung einen Bausatz zur Versorgung von Kopffrakturen des Humerus vor, der wenigstens einen erfindungsgemäßen Kurznagel-Stützkörper sowie mindestens eine ein Befestigungselement bildende Fixationsschraube enthält. Besonders zweckmäßig umfaßt der Bausatz wenigstens einen Kurznagel-Stützkörper sowie mindestens eine dem genannten Kurznagel zugeordnete Gruppe von mehreren, zweckmäßig vier Fixationsschrauben.

Weitere bevorzugte Merkmale, Ausführungsformen und Möglichkeiten der Erfindung gehen aus den Unteransprüchen und der Beschreibung hervor. Besonders bevorzugte Ausführungsbeispiele werden anhand der Zeichnung näher erläutert. In der Zeichnung zeigen
- Fig. 1: medial in Vorderansicht einen erfindungsgemäßen Humerus- Knochennagel,
- Fig. 2: im Längsschnitt II-II den Knochennagel gemäß Figur 1,
- Fig. 3: lateral in Rückansicht den Knochennagel gemäß Figur 1,
- Fig. 4: einen Ausschnitt A gemäß Figur 3 in starker Vergrößerung,
- Fig. 5: einen Ausschnitt B gemäß Figur 2 in starker Vergrößerung und
- Fig. 6 und 7: in Ansicht von lateral und dorsal bzw. ventral einen in den Hume- rus eingebrachten erfindungsgemäßen Humerus-Knochennagel mit Fixationsschrauben.

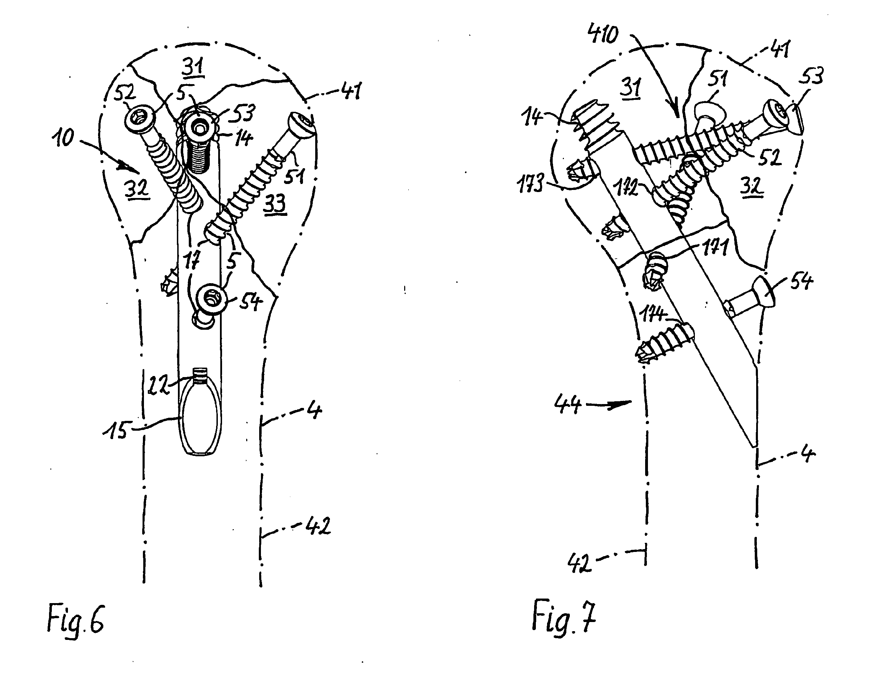

Wie insbesondere aus Fig. 6 und 7 ersichtlich, sind die im folgenden beschriebenen Knochennägel 10 zur Versorgung von Kopffrakturen des Humerus 4 ausgebildet und vorgesehen. Der Humerus 4 weist drei Knochensegmente auf, nämlich den Kopfteil 41 am proximalen Ende, den nicht dargestellten Fußteil am distalen Ende sowie einen das Kopfteil 41 mit dem Fußteil verbindenden Schaft 42.

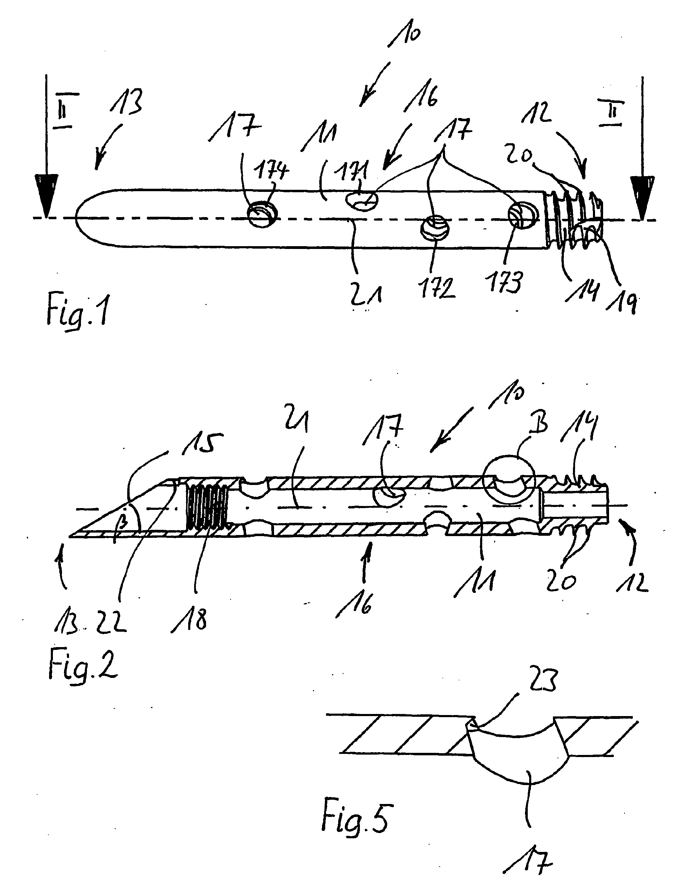

Der Humerus-Knochennagel 10 gemäß Fig. 1 besteht aus einem kreiszylinderförmigen, hohlen Stützkörper 11 mit proximalem Ende 12 und distalem Ende 13. Der Stützkörper 11 ist linear, d.h. axial gerade ausgebildet und weist an seinem proximalen Ende 12 ein Außengewinde 14 auf. An seinem distalen Ende 13 ist der Stützkörper 11 mit einer Schräge 15 versehen. Des weiteren sind im Mittelabschnitt 16 zwischen dem proximalen Ende 12 und dem distalen Ende 13 mehrere Durchgangsbohrungen 17 vorgesehen. Im Bereich des distalen Endes 13 ist weiterhin ein Innengewinde 18 ausgebildet, das insbesondere zur lösbaren Befestigung eines (nicht dargestellten) Zielgerätes, eines Adapters für einen Schraubendreher oder dergleichen Ansatzteile dient.

Der üblicherweise aus nichtrostendem Stahl und vorzugsweise aus Titan hergestellte Humeruskopf-Stützkörper 11 weist eine Länge von ca. 70 mm bis 90 mm, vorzugsweise ca. 70 mm auf. Im übrigen kann je nach Anwendungsfall die Länge sowohl nach oben als auch nach unten variieren. Entscheidend bei der Wahl der Länge des Stützkörpers 11 ist es, daß sich der Stützkörper 11 in eingesetztem Zustand innerhalb des Humerus 4 lediglich über den Kopfteil 41 erstreckt. Der Außendurchmesser des Stützkörpers 11 beträgt zwischen etwa 6 und 10 mm. Besonders bevorzugt ist ein Durchmesser von etwa 8 mm. Vorteilhaft beträgt der maximale Durchmesser 10 mm. Insbesondere kann zweckmäßig ein dem Knochennagel 10 für den Humeruskopf entsprechender Nagel für den Humerus-Fußteil vorgesehen werden.

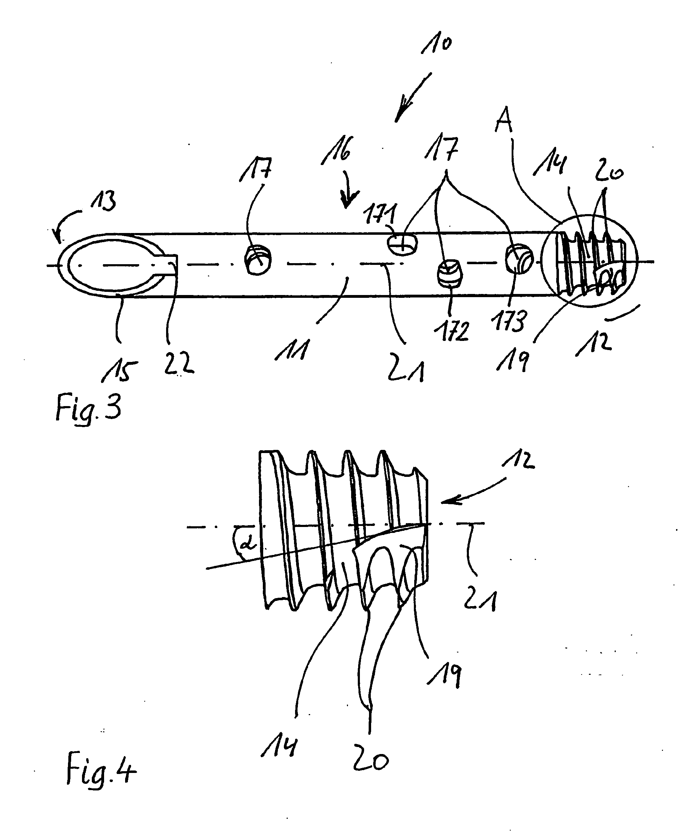

Das Außengewinde 14 ist als selbstschneidendes Gewinde ausgebildet und weist hierzu ein Kerbe 19 auf (siehe insbesondere Fig. 4). Die Kerbe 19 erstreckt sich in der gezeigten Ausführungsform über zwei vollständige Gewindegänge 20. Alternativ ist es jedoch auch denkbar, daß sich die Kerbe 19 über einen einzigen Gewindegang 20 bzw. über mehr als zwei Gewindegänge 20 erstreckt. Die Ausrichtung der Kerbe 19 verläuft gekrümmt mit einem mittleren kleinen Winkel a zur Mittelachse 21 des Knochennagels 11. Der Winkel a ist jedoch variabel.

Die Steigung des Außengewindes 14 ist so gewählt, daß einerseits eine ausreichende Halterung und Fixierung im Knochen gewährleistet ist. Das bedeutet, daß ein möglichst großer Spanraum zwischen den Gewindegängen zur Aufnahme von Knochensubstanz vorhanden ist. Dies ist bei großen Steigungen der Fall. Andererseits ist das Gewinde derart ausgebildet, daß eine Feinjustierung möglich ist. Hierfür muß die Steigung ausreichend klein sein, so daß bei einer Drehung um einen bestimmten Winkel lediglich ein geringer Vorschub erzielt wird. Eine bevorzugte Steigung, die die genannten Anforderungen besonders gut erfüllt, weist den Wert 1,75 auf. Andere kleinere und größere Steigungen sind jedoch je nach Anwendungsfall ebenfalls möglich.

Die Schräge 15 am distalen Ende 13 des Stützkörpers 11 weist einen Winkel β von etwa 30° zur Mittelachse 21 auf. Der Wert dieses Winkels ist variabel bzw. in anderer Größe wählbar. Allerdings muß sichergestellt sein, daß sich die Schräge 15 an die Formgebung bzw. Außenkontur des Knochens derart anpaßt, daß der Stützkörper 11 in eingesetztem Zustand nahezu bündig mit dem Knochen 4 abschließt. Dies wird insbesondere aus Fig. 7 deutlich. In diesem Zusammenhang ist auch die Steigung des Außengewindes 14 von Bedeutung. Denn um den wenigstens nahezu bündigen Abschluß des Stützkörpers 11 an der Außenseite des Knochens 4 zu ermöglichen, ist u.U. eine letzte Drehung des Stützkörpers 11 um bis zu 360° erforderlich. Bei einer zu großen Steigung würde der Stützkörper 11 dann wegen des großen Vorschubs aus dem Knochen 4 austreten, was jedoch in jedem Fall zu verhindern ist.

Die Wahl der Schräge 15 hängt wesentlich vom dem Winkel ab, in dem der Knochennagel 10 bzw. der Stützkörper 11 in den Knochen 4 eingeführt wird. Im Bereich der Schräge 15 ist parallel zur Mittelachse 21 eine Nut 22 vorgesehen. Diese Nut 22 dient neben der Schräge 15 als formschlüssige Arretierung zum drehfesten Anschluß des Stützkörpers 11 an weitere chirurgische Geräte, insbesondere Einführhilfen für den Stützkörper 11 sowie Zielgeräte. An den Stützkörper 11 wird üblicherweise ein Verlängerungsstück angebracht, das eine korrespondierende Schräge sowie einen Vorsprung aufweist, der in die Nut 22 greift. Durch eine innen geführte Schraube werden der Stützkörper 11 und das Verlängerungsstück miteinander verbunden, wobei die Schraube in ein Innengewinde 18 greift. Durch die Drehung des Verlängerungsstücks wird somit zwangsläufig auch der Stützkörper 11 gedreht und damit in eine vorbereitete Bohrung eingedreht.

Im Ausführungsbeispiel gemäß Fig. 1 sind im Bereich des Mittelabschnittes 16 vier Bohrungen 17 angeordnet. Bei diesen Bohrungen 17 handelt es sich um mit nachstehend beschriebener Ausnahme gewindefreie Durchgangslöcher, die zur Aufnahme von Befestigungselementen, insbesondere von aus Fig. 6 und 7 ersichtlichen Fixationsschrauben 5, dienen. Die Anordnung und Lage bzw. Ausrichtung der Durchgangsbohrungen 17 werden nach Maßgabe von statistisch ermittelten, am häufigsten auftretenden Bruchlinien des Knochenteils 41 ausgewählt, derart, daß in die Durchgangsbohrungen 17 einzuführende Fixationsschrauben 5 jeweils mit zugehöriger Bruchlinie bzw. Endstellen-Bruchfläche zwischen Frakturteilen 31, 32 bzw. 33 einen weitgehend annähernd oder wenigstens, im wesentlichen rechten Winkel bilden. Diese Ausbildung und Anordnung eines Humeruskopf-Kurznagels 10 mit den Fixationsschrauben 5 ist insbesondere aus Fig. 6 und 7 ersichtlich. In den Ausführungsformen verlaufen die Mittelachsen der Durchgangsbohrungen 17 sämtlich in einem Winkel zur Mittelachse 21, der größer oder kleiner als 90° ist.

Die in Fig. 1 bis 3 sowie in Fig. 6 und 7 im proximalen Abschnitt liegenden Durchgangsbohnungen 17 bilden jeweils eine Gruppe von drei Durchgangsbohrungen 171, 172, 173, die so angeordnet und orientiert sind, daß die drei zugeordneten Fixationsschrauben 5 in der beschriebenen Art jeweils eine zugehörige Endstellen-Bruchfläche im wesentlichen rechtwinklig durchgreifen, wobei sie sich zum größten Teil in einem nach innen weiten lateralseitigen Bereich 410 des Humeruskopfes 41 erstrecken. Entsprechend ist der Kurznagel-Stützkörper 11 so dimensioniert und angeordnet, daß er sich, ausgehend von seinem distalen Ende, d.h. von seiner im Bereich des oder unmittelbar unterhalb des chirurgischen Halses (Collum chirurgicum) 44 liegenden lateralen Schräge 15 in den medialen Bereich des Humeruskopfes 41 hinein erstreckt. Im distalen Abschnitt des Stützkörpers 11 ist die vierte Durchgangsbohrung 174 ausgebildet, die eine relativ kurze, den Humeruskopf 41 im Bereich seines chirurgischen Halses durchquerende, keine Bruchstelle durchgreifende Fixationsschraube 54 aufnimmt. Im Ausführungsbeispiel sind die Durchgangsbohrungen 171, 172 und 173 bzw. die Fixationsschrauben 51, 52 und 53 nach Maßgabe der Frakturlinien relativ steil zur Mittelachse 21 des Stützkörpers 11 gerichtet, wobei sie berührungslos in Überschneidung nebeneinander und übereinander zu liegen kommen.

Die Lage bzw. Ausrichtung einzelner oder sämtlicher Durchgangsbohrungen 17 sind jedoch nicht auf die gezeigte und beschriebene Darstellung beschränkt. Vielmehr können Durchgangsbohrungen jeder Art und Lage auch unabhängig von den statistisch am häufigsten Bruchlinien vorgesehen werden. Die Anzahl der Durchgangsbohrungen 17 ist auch nicht auf vier beschränkt. Es sind Anwendungsfälle vorstellbar, in denen lediglich eine Durchgangsbohrung 17 vorgesehen ist. Die Durchgangsbohrungen 17 können unterschiedliche Dürchmesser aufweisen. Bevorzugt beträgt der Durchmesser aller Bohrungen 17 im Ausführungsbeispiel etwa 3 bis 4 mm, insbesondere ca. 3,7 mm.

In dem Ausführungsbeispiel der Fig. 1 bis 7 weist jede Durchgangsbohrung 17 an einer Seite einen Absatz 23 auf. Dieser ist durch einen Gewindeansatz gebildet und dient dazu, das Durchrutschen der Befestigungselemente 5 zu verhindern und diese auch unverlierbar zu halten. Befestigungselemente, die als Fixationsschrauben 5 ausgebildet sind, werden, in Eingriff mit dem Gewindeansatz, durch Drehung in die Durchgangsbohrungen 17 eingeführt. Es besteht auch die Möglichkeit, daß eine oder jede Durchgangsbohrung 17 auf beiden Seiten einen Absatz 23 aufweist. Die Absätze 23 begrenzen die Befestigungselemente in ihrer linearen (axialen) Bewegung z.B. dann, wenn die Fixationsschrauben keinen ausreichenden Halt im Knochen 41 haben. Mit dem Absatz 23 werden die Fixationsschrauben 5 dennoch in ihrer Stützposition gehalten.

Vom distalen Ende 13 des Stützkörpers 11 aus gesehen hinter der Schräge 15 ist das Innengewinde 18 vorzugsweise als M6-Gewinde ausgebildet. Je nach Durchmesser des Stützkörpers 11 bzw. nach Anforderung kann jedoch ein anderes Gewindemaß verwendet werden.

In weiteren nicht dargestellten Ausführungsformen kann der Stützkörper 11 weniger oder mehr als vier Bohrungen aufweisen. Auch ist die Anordnung bzw. Lage der Bohrungen variabel bzw. frei wählbar, so daß eine optimale Fixation in Abhängigkeit von den und nach Maßgabe der jeweiligen Bruchlinien gewährleistet werden kann. Der Stützkörper 11 kann auch aus anderen als den oben genannten Materialien bestehen. Insbesondere ist eine Herstellung aus hochfestem Kunststoff oder dergleichen möglich.

Üblicherweise wird der der Stützkörper 11 des Knochennagels 10 in eine vorbereitete Bohrung mit seinem proximalen Ende 12 voraus in den Knochen 41 eingeführt, wobei Vortrieb durch das selbstschneidende Außengewinde 14 erfolgt. So kann auf ein Vorschneiden eines Gewindes verzichtet werden. Das Einbringen des Knochennagels 10 in den Knochen erfolgt z.B. mittels einer Verlängerung, die einen Adapter bildet, über einen Führungsspieß. Dazu wird ein geeigneter Schraubendreher verwendet. Mittels eines Zielgerätes werden dann die Bohrlöcher für die Fixationsschrauben markiert und gebohrt.

Der erfindungsgemäße Knochennagel 10 eignet sich besonders zur Bildung eines erfindungsgemäßen Bausatzes. Dieser umfaßt für die Ausführungsbeispiele gemäß Fig. 1 bis 7 wenigstens einen Kurznagel-Stützkörper 11 sowie wenigstens eine Gruppe von vier Fixationsschrauben 51 bis 54 für die beschriebenen Durchgangsdurchbohrungen 171 bis 174. Vorteilhaft ist der Bausatz mit mehreren erfindungsgemäßen Kurznagel-Stützkörpern 11 bestückt, die, nach Maßgabe von statistisch ausgewerteten Frakturen, unterschiedliche Bohrungsmuster, also Anordnungen und Ausrichtungen der Durchgangsdurchbohrungen 17 und/oder unterschiedliche Längen aufweisen. Einem solchen Bausatz werden geeignete Befestigungselemente hinzugefügt, die in der Zahl kleiner als die Gesamtzahl der Bohrungen 17 der Stützkörper 11 sein können. Zu diesem Zweck werden mehrere Stützkörper 11 in der Weise einheitlich ausgebildet, daß für Befestigungselemente mit gleichem Durchmesser die Durchmesser der Durchgangsbohrungen 17 gleich sind.

## Patentansprüche

1. Humerus-Knochennagel (10) zum Versorgen von Frakturen (31,32,33) des proximalen Kopfteils (41) des den Kopfteil (41) und den Humerusschaft (42) als Knochensegmente aufweisenden Humerus (4), wobei der Humerus-Knochenna gel (10) einen stabförmigen, insbesondere rohrförmigen Stützkörper (11) aufweist, wobei der Stützkörper (11) als sich im Wesentlichen linear erstreckender Kurznagel mit proximalem Ende (12) und distalem Ende (13) derart kurz ausgebildet ist, dass er sich in in den Humerus (4) eingeführtem Zustand von dem distalen Ende (13) zu dem proximalen Ende (12) lediglich über den proximalen Humeruskopfteil (41) erstreckt, wobei der Kurznagel-Stützkörper (11) an seinem proximalen Ende (12) ein AuBengewinde (14) zur Fixierung und Stützung innerhalb des Knochens aufweiset und in einem Mittelabschnitt (16) des Kurznagel-Stützkörpers (11) zwischen dem distalen Ende (13) und dem proximalen Ende (12) mindestens eine Durchgangsbohrung (17) zur Aufnahme eines stiffartigen Befestigungselements (5) vorgesehen ist, wobei die mindestens eine odes wenigstens eine der Durchgangsbohrungen (17) derart ausgebildet ist, dass das korrespondierende, durch die Durchgangsbohrung (17) hindurchgesetzte Befestigungselement (5) in seiner linearen Bewegung begrenzt ist und wobei das proximale Außengewinde (14) selbstschneidend ausgebildet ist..

2. Knochennagel nach Anspruch 1, **dadurch gekennzeichnet, dass** das proximale Außengewinde(14) mit einer solchen Steigung versehen ist, die zum Einen eine ausreichende Halterung und Fixierung im Knochen gewährleistet und zum Anderen eine Feinjustierung zulässt.

3. Knochennagel nach Anspruch 1 oder 2, **dadurch gekennzeichnet, dass** die Steigung des proximalen Außengewindes (14) im Wertebereich von ca. 1 bis 2,5 liegt und vorzugsweise ca. 1,75 beträgt.

4. Knochennagel nach einem der Ansprüche 1 bis 3, **dadurch gekennzeichnet, dass** der Kurznagel-Stützkörper (11) an seinem distalen Ende (13) mit einem Innengewinde (18), insbesondere zur Verbindung mit einem Zielgerät, versehen ist.

5. Knochennagel nach einem der Ansprüche 1 bis 4, **dadurch gekennzeichnet, dass** an dem distalen Ende (13) des Kurznagel-Stützkörpers (11) eine Schräge (15) vorgesehen ist, die zum wenigstens nahezu bündigen Abschluss mit dem Humerus (4) im Wesentlichen von der schrägen Winkellage des Kucznagel-Stützkörpers (11) abhängt.

6. Knochennagel nach Anspruch 5, **dadurch gekennzeichnet, dass** im Bereich der Schräge (15) eine Nut (22) zum drehfesten Anschluss des Kurznagel-Stützkörpers (11) an ein chirurgisches Gerät angeordnet ist.

7. Knochennagel nach einem der Ansprüche 1 bis 6, **dadurch gekennzeichnet, dass** im Bereich des Mittelabschnittes (16) des Kurznagel-Stützkörpers (11) zur Versorgung von Kopffrakturen (31, 32, 33) des Humerus (4) wenigstens drei und insbesondere vier Durchgangsbohrungen (17) angeordnet und ausgebildet sind.

8. Knochennagel nach einem der Ansprüche 1 bis 7, **dadurch gekennzeichnet, dass** mehrere Durchgangsbohrungen (17) vorgesehen sind, die in jedem gewünschten Winkel zueinander und/oder zur Mittelachse (21) des Kurznagel-Stützkörpers (11) ausgebildet sind.

9. Knochennagel nach einem der Ansprüche 1 bis 8, **dadurch gekennzeichnet, dass** im Bereich einer Fraktur die Lage wenigstens einer, vorzugsweise jeder Durchgangsbohrung (17) entsprechend den häufigsten Bruchlinien des proximalen Humerus-Kopfteils (41) ausgewählt ist, derart, dass die in die Durchgangsbohrungen (17) einführbaren Befestigungselemente (5) oder dergleichen jeweils mit einer zugeordneten Bruchlinie oder mehreren zugeordneten Bruchlinien einen im Wesentlichen rechten Winkel bilden.

10. Knochennagel nach Anspruch 9, **dadurch gekennzeichnet, dass** im proximalen Abschnitt des Kurznagel-Stützkörpers (11) liegende Durchgangsbohrungen (17) eine Gruppe von wenigstens zwei Durchgangsbohrungen (171, 172, 173) bilden, die so angeordnet und orientiert sind, dass jeweils der im Wesentlichen rechte Winkel zwischen Befesfigungselement (51, 52, 53) und zugeordncter Bruchlinie gebildet ist, und dass im distalen Abschnitt des Kurznagel-Stützkörpers (11) wenigstens eine weitere Durchgangsbohrung (174) zur Aufnahme eines Befestigungselements (54) außerhalb einer Kopffraktur in dem Lagebereich des Kurznagel-Stützkörpers (11), der dem chirurgischen Hals (44) des Humeruskopfes (41) entspricht, ausgebildet ist.

11. Knochennagel nach einem der Ansprüche 1 bis 10, **dadurch gekennzeichnet, dass** der Kurznagel-Stützkörper (11), ausgehend von seinem insbesondere mit Schräge (15) versehenen distalen Ende, in dem medialen Bereich des Humerus-Kopfteils (41) zu liegen kommt

12. Knochennagel nach einem der Ansprüch 1 bis 11, **dadurch gekennzeichnet, dass** die mindestens eine oder wenigstens eine der Durchgangsbohrungen (17) an mindestens einer ihrer Endseiten einen Absatz (23) aufweist, derart, dass das korrespondierende, durch die Durchgangsbohrung (17) hindurchgesetzte stiftartige Befestigungselement (5) in seiner linearen Bewegung begrenzt ist.

13. Bausatz zur Versorgung von Kopffrakturen des Humerus zur Bildung wenigstens eines Knochennagels (10) nach einem der Ansprüche 1 bis 12, bestehend aus wenigstens einem Kurznagel-Stützkörper (11) sowie mindestens einer ein Befestigungselement (5) bildenden Fixationsschraube.

14. Bausatz nach Anspruch 13, **dadurch gekennzeichnet , dass** er aus wenigstens einem Kurznagel-Stützkkörper (11) sowie mindestens einer dem genannten Knochennagel (10) zugeordneten Gruppe von mehreren, vorzugsweise vier Fixationssehrauben (5) besteht.

## Claims

1. Humerus bone nail (10) for treating fractures (31, 32, 33) of the proximal head portion (41) of the humerus (4) which comprises the head portion (41) and the humerus shaft (42) as bone segments, wherein the humerus bone nail (10) has a rod-shaped, in particular tubular supporting body (11), wherein the supporting body (11) is a short nail extending essentially linearly with proximal end (12) and distal end (13), said supporting body (11) being constructed in such a short manner that in the state introduced into the humerus (4) it extends from the distal end (13) to the proximal end (12) only over the proximal head portion (41) of the humerus, wherein the short-nail supporting body (11) has at its proximal end (12) an external thread (14) for fixing and support inside the bone, and in a centre section (16) of the short-nail supporting body (11) between the distal end (13) and the proximal end (12) is provided at least one through-bore (17) for receiving a pin-like fastening element (5), wherein the at least one or at least one of the through-bores (17) is constructed in such a way that the corresponding fastening element (5), which is passed through the through-bore (17), is limited in its linear movement, and wherein the proximal external thread (14) is self tapping.

2. Bone nail according to claim 1, **characterised in that** the proximal external thread (14) is provided with such a pitch as firstly to ensure sufficient holding and fixing in the bone and secondly to permit fine adjustment.

3. Bone nail according to claim 1 or 2, **characterised in that** the pitch of the proximal external thread (14) is within the range of values of approximately 1 to 2.5 and preferably approximately 1.75.

4. Bone nail according to any one of claims 1 to 3, **characterised in that** the short-nail supporting body (11) is provided at its distal end (13) with an internal thread (18), in particular for connection to a target device.

5. Bone nail according to any one of claims 1 to 4, **characterised in that** at the distal end (13) of the short-nail supporting body (11) is provided a chamfer (15) which, for ending at least nearly flush with the humerus (4), depends essentially on the oblique angular position of the short-nail supporting body (11).

6. Bone nail according to claim 5, **characterised in that** in the region of the chamfer (15) is arranged a groove (22) for non-rotatable connection of the short-nail supporting body (11) to a surgical instrument.

7. Bone nail according to any one of claims 1 to 6, **characterised in that** in the region of the centre section (16) of the short-nail supporting body (11) are arranged and formed at least three and in particular four through-bores (17) for supporting head fractures (31, 32, 33) of the humerus (4).

8. Bone nail according to any one of claims 1 to 7, **characterised in that** several through-bores (17) are provided, which are formed at any desired angle to each other and/or to the centre axis (21) of the short-nail supporting body (11).

9. Bone nail according to any one of claims 1 to 8, **characterised in that** in the region of a fracture the position of at least one, preferably each through-bore (17) is selected according to the commonest fracture lines of the proximal head portion (41) of the humerus, such that the fastening elements (5) or the like, which can be introduced into the through-bores (17), each form an essentially right angle with an associated fracture line or with several associated fracture lines.

10. Bone nail according to claim 9, **characterised in that** through-bores (17) located in the proximal section of the short-nail supporting body (11) form a group of at least two through-bores (171, 172, 173) which are arranged and oriented in such a way that in each case the essentially right angle between fastening element (51, 52, 53) and associated fracture line is formed, and **in that** in the distal section of the short-nail supporting body (11) is formed at least one further through-bore (174) for receiving a fastening element (54) outside a head fracture in the mounting region of the short-nail supporting body (11) which corresponds to the surgical neck (44) of the head (41) of the humerus.

11. Bone nail according to any one of claims 1 to 10, **characterised in that** the short-nail supporting body (11), starting from its distal end which is in particular provided with a chamfer (15), comes to lie in the medial region of the head portion (41) of the humerus.

12. Bone nail according to any one of claims 1 to 11, **characterised in that** the at least one or at least one of the through-bores (17) has a heel (23) at at least one of its end faces, such that the corresponding pin-like fastening element (5) which is passed through the through-bore (17) is limited in its linear movement.

13. Kit for supporting head fractures of the humerus for forming at least one bone nail (10) according to any one of claims 1 to 12, consisting of at least one short-nail supporting body (11) as well as at least one fixation screw which forms a fastening element.

14. Kit according to claim 13, **characterised in that** it consists of at least one short-nail supporting body (11) as well as at least one group of several, preferably four fixation screws (5) associated with said bone nail (10).

## Revendications

1. Clou (10) destiné à l'os humérus en vue de réparer des fractures (31, 32, 33) de la partie de tête proximale (41) de l'humérus (4) dont les segments osseux sont la partie de tête (4) et la hampe d'humérus (42), le clou d'humérus (10) possédant un corps de support (11) en forme de barre, notamment en forme de tube, le corps de support (11) ayant une conformation courte se présentant sous la forme d'un clou court qui s'étend sensiblement linéairement et qui comporte une extrémité proximale (12) et une extrémité distale (13) de sorte que, lorsqu'il est inséré dans l'humérus, il s'étend de l'extrémité distale (13) à l'extrémité proximale (12) seulement sur la partie de tête proximale (41) de l'humérus, le corps de support (11) du clou court possédant à son extrémité proximale (12) un filetage extérieur (14) destiné à la fixation et au support à l'intérieur de l'os et au moins un perçage traversant (17) étant ménagé dans une portion médiane (16) du corps de support de clou court (11), entre l'extrémité distale (13) et l'extrémité proximale (12), afin de recevoir un élément de fixation (5) de type broche, l'au moins un ou l'un au moins des perçages traversants étant conformé de telle sorte que l'élément de fixation (5) correspondant qui est passé à travers le perçage traversant (17) est limité dans son déplacement linéaire et le filetage extérieur proximal (14) étant conformé pour être autotaraudeur.

2. Clou selon la revendication 1, **caractérisé en ce que** le filetage extérieur proximal (14) est doté d'un pas qui garantit d'une part un support suffisant et une fixation suffisante dans l'os et qui permet d'autre part un ajustement fin.

3. Clou selon la revendication 1 ou 2, **caractérisé en ce que** le pas du filetage extérieur proximal (14) est de l'ordre de 1 à 2,5 environ et est avantageusement égal à 1,75 environ.

4. Clou selon l'Une des revendications 1 à 3, **caractérisé en ce que** le corps de support de clou court (11) est doté à son extrémité distale (13) d'un filetage intérieur (18), notamment pour la liaison à un appareil de visée.

5. Clou selon l'une des revendications 1 à 4, **caractérisé en ce qu'**il est prévu, à l'extrémité distale (13) du corps de support de clou court (11), un biseau qui dépend sensiblement de la position angulaire inclinée du corps de support de clou court (11) de façon à venir à peu près à fleur de l'humérus (4).

6. Clou selon la revendication 5, **caractérisé en ce qu'**une gorge (22) est ménagée dans la région du biseau (15) en vue de raccorder de façon fixe en rotation le corps de support de clou court (11) à un appareil chirurgical.

7. Clou selon l'une des revendications 1 à 6, **caractérisé en ce que** au moins trois et notamment quatre perçages traversants (17) sont disposés et ménagés dans la région de la portion médiane (16) du corps de support de clou court (11) en vue de traiter des fractures de tête (31, 32, 33) de l'humérus (4).

8. Clou selon l'une des revendications 1 à 7, **caractérisé en ce qu'**il est prévu plusieurs perçages traversants (17) qui sont ménagés suivant chaque angle souhaité l'un par rapport à l'autre et/ou par rapport à l'axe médian (21) du corps de support de clou court (11).

9. Clou selon l'une des revendications 1 à 8, **caractérisé en ce que** dans la région d'une fracture la position d'au moins un, avantageusement de chaque, perçage traversant (17) est choisie de façon à correspondre aux lignes de fracture les plus fréquentes de la partie de tête proximale (41) de l'humérus de sorte que les éléments de fixation (5), insérables dans les perçages traversants (17), ou analogues forment chacun un angle sensiblement droit avec une ligne de fracture associée ou plusieurs lignes de fracture associées.

10. Clou selon la revendication 9, **caractérisé en ce que** des perçages traversants situés dans la portion proximale du corps de support de clou court (11) forment un groupe d'au moins deux perçages traversants (171, 172, 173) qui sont disposés et orientés de telle sorte que l'angle sensiblement droit est formé à chaque fois entre un élément de fixation (51, 52, 53) et la ligne de fracture associée, et **en ce que** au moins un autre perçage traversant (174) est ménagé dans la portion distale du corps de support de clou court (11) en vue de recevoir un élément de fixation (54) en dehors d'une fracture de tête dans la région de position du corps de support de clou court (11) qui correspond au col chirurgical (44) de la tête (41) de l'humérus.

11. Clou selon l'une des revendications 1 à 10, **caractérisé en ce que** le corps de support de clou court (11) vient se placer, à partir de son extrémité distale dotée notamment d'un biseau (15), dans la région médullaire de la partie de tête (41) de l'humérus.

12. Clou selon l'une des revendications 1 à 11, **caractérisé en ce que** l'au moins un ou l'un au moins des perçages traversants (17) comporte sur au moins un de ses côtés d'extrémité un talon (23) de sorte que l'élément de fixation (5) correspondant qui est passé à travers le perçage traversant (17) est limité dans son déplacement linéaire.

13. Kit de réparation de fractures de tête de l'humérus pour former au moins un clou (10) selon l'une des revendications 1 à 12, constitué d'au moins un corps de support de clou court (11) ainsi que d'au moins une vis de fixation formant un élément de fixation (5).

14. Kit selon la revendication 13, **caractérisé en ce qu'**il est constitué d'au moins un corps de support de clou court (11) ainsi que d'au moins un groupe de plusieurs, avantageusement quatre, vis de fixation (5) qui est associé audit clou (10).
